# EUROPÄISCHE PATENTSCHRIFT

(11) **EP 3 476 358 B1**
(45) Veröffentlichungstag und Bekanntmachung des Hinweises auf die Patenterteilung: **01.07.2020**
(21) Anmeldenummer: 17198926.2
(22) Anmeldetag: 27.10.2017
(51) Int. Cl.: A61B 34/20, A61B 34/30, A61B 17/00, A61B 90/00

(54) **SYSTEM ZUM NACHVERFOLGEN EINER POSITION EINES ZIELOBJEKTS**
SYSTEM FOR TRACKING A POSITION OF A TARGET OBJECT
SYSTÈME DE SUIVI D'UNE POSITION D'UN OBJET CIBLE

(43) Veröffentlichungstag der Anmeldung: 01.05.2019
(73) Patentinhaber: Siemens Healthcare GmbH, 91052 Erlangen (DE)
(72) Erfinder: MEWES, Philip, 90419 Nürnberg (DE); MÜLLER, Gunter, 90562 Heroldsberg (DE)

(56) Entgegenhaltungen:
- EP-A1- 1 854 425
- WO-A1-2007/003949
- WO-A1-2016/168671
- US-A1- 2014 188 132

## Beschreibung

Die Erfindung betrifft ein, insbesondere robotisches, System zum Nachverfolgen einer Position eines Zielobjekts. Die Erfindung kann bevorzugt in einem medizinischen Umfeld, beispielsweise in der minimalinvasiven Chirurgie, angewendet werden. Hier kann das Zielobjekt dann insbesondere ein zu behandelnder oder zu untersuchender Patient sein.

Aus der Praxis ist es bekannt, einen Marker an dem Patienten anzuordnen und anhand dieses Markers die Position des Patienten mittels eines Navigationssystems, welches beispielsweise eine auf den Marker gerichtete Kamera umfassen kann, nachzuverfolgen. Nach einer Registrierung beispielsweise mit von einem Röntgensystem erzeugten Bilddaten des Patienten kann so auch bei einer Bewegung des Patienten in Echtzeit eine ortsgenaue Überlagerung, beispielsweise als Overlay auf einem medizinischen Bildschirm angezeigt werden. Insbesondere bei einer Verwendung zusätzlicher Werkzeuge oder Geräte können dabei schnell beengte Platzverhältnisse entstehen. Zudem kann ein signifikanter Aufwand entstehen, um jeweilige räumliche Lagebeziehungen zwischen allen beteiligten beweglichen Geräten und Objekten zu überwachen und zueinander konsistent zu halten.

Aus der WO 2016 / 168 671 A1 ist ein Verfahren zu Ausführen einer robotisch unterstützten Operation bekannt. Darin wird ein Roboterarm relativ zu einem Patienten und einem bildgebenden Gerät in eine vorbestimmte Position bewegt basierend auf Bilddaten des Patienten. Der Roboterarm hält einen Endeffektor in der vorbestimmten Position in Bezug auf den Patienten und kollidiert dabei nicht mit dem bildgebenden Gerät oder mit dem Patienten wenn das bildgebende Gerät relativ zu dem Patienten bewegt wird.

Die EP 1 854 425 A1 offenbart ein Verfahren zur Bestimmung der Position einer medizinischen Einrichtung oder eines Patientenkörperteils. Dabei werden mit zwei separaten Positionserfassungseinrichtungen zwei Positionserfassungen durchgeführt, denen dann eine bestimmte Priorität zugeordnet wird, welche die Gewichtung der Positionserfassungen definiert. Die Positionsbestimmung erfolgt dann unter Berücksichtigung der Gewichtung aus einer Kombination der Positionserfassungen.

Die WO 2007 / 003 949 A1 beschreibt einen Roboter mit einem Bilderfassungsgerät zum Erfassen von Bildern von Markern und einem Indikator, der an einem Werkstück angeordnet ist. Durch Bestimmung einer Position der Marker aus den Bildern wird eine Position des Werkstücks bestimmt. Wenn die Marker verdeckt sind, soll bei einer Bewegung des Indikators die neue Position des Indikators bestimmt werden und der Roboter weiter eine vorbestimmte Bewegung relativ zu dem Werkstück ausführen.

Aus der US 2014 / 0 188 132 A1 ist ein robotisches Operationssystem bekannt, umfassend eine mobile Basis, eine erste und zweite bewegliche Unterstützungsstruktur, eine Strahlquelle und einen mit dieser gekoppelten Detektor sowie ein Operationsinstrument. Die erste Unterstützungsstruktur koppelt die Strahlquelle oder den Detektor mit der mobilen Basis. Die zweite Unterstützungsstruktur koppelt das Operationsinstrument mit einem Verbindungselement zwischen der Strahlquelle und dem Detektors. Die zweite Unterstützungsstruktur umfasst einen gesteuerten Aktuator, zum elektromechanischen Charakterisieren einer Bewegung des Operationsinstruments relativ zu dem Verbindungselement.

Aufgabe der vorliegenden Erfindung ist es, auf besonders einfache Weise ein Nachverfolgen einer Position (Tracking) eines Zielobjekts mit verbesserter Zuverlässigkeit zu ermöglichen.

Diese Aufgabe wird erfindungsgemäß durch den Gegenstand des unabhängigen Patentanspruchs gelöst. Vorteilhafte Ausgestaltungen und Weiterbildungen der Erfindung sind in den abhängigen Patentansprüchen sowie in der Beschreibung und in den Zeichnungen angegeben.

Ein erfindungsgemäßes, insbesondere robotisches, System weist einen Roboter mit einem beweglichen Roboterarm, eine Nachverfolgungseinrichtung und eine Datenverarbeitungseinrichtung auf. Die Nachverfolgungseinrichtung dient, ist also eingerichtet, zum Nachverfolgen einer Position eines Zielobjekts, insbesondere eines Patienten, anhand eines an dem Zielobjekt angeordneten Markers durch Verarbeiten von von der Nachverfolgungseinrichtung und/oder dem Marker bereitgestellten Daten mittels der Datenverarbeitungseinrichtung des Systems. Um das Nachverfolgen der Position des Zielobjekts, also des Zielobjekts, auf besonders einfache und platzsparende Weise zu ermöglichen, ist die Nachverfolgungseinrichtung zumindest mittelbar an dem Roboterarm befestigt. Um bei dem Nachverfolgen der Position des Zielobjekts, also des Zielobjekts, eine verbesserte Zuverlässigkeit zu erzielen, weist das System zusätzlich wenigstens Kontrollmarker auf, der in einer vorgegebenen, also bei einem Betrieb des Roboters bekannten, räumlichen Lagebeziehung zu der Nachverfolgungseinrichtung angeordnet ist.

Der Kontrollmarker ist dabei in einer bei dem Betrieb des Roboters konstanten räumlichen Lagebeziehung zu der Nachverfolgungseinrichtung angeordnet. In einer bevorzugten Weiterbildung ist der Kontrollmarker, zumindest mittelbar, an dem Roboterarm befestigt. Ebenso wie der Kontrollmarker kann auch die Nachverfolgungseinrichtung in einer, wenigstens bei dem Betrieb des Roboters oder der Bewegung des Roboterarms, konstanten, vorgegebenen räumlichen Lagebeziehung zu dem Roboterarm an diesem befestigt, also angeordnet sein.

Das System ist dazu eingerichtet, bei dem Nachverfolgen des Zielobjekts die räumliche Lagebeziehung zwischen der Nachverfolgungseinrichtung und dem Kontrollmarker, insbesondere kontinuierlich oder regelmäßig, zu messen oder zu bestimmen. Das System ist weiterhin dazu eingerichtet, die gemessene räumliche Lagebeziehung mit der realen, vorgegebenen räumlichen Lagebeziehung zwischen der Nachverfolgungseinrichtung und dem Kontrollmarker zu vergleichen. Diese vorgegebene räumliche Lagebeziehung ist also bekannt, da eine jeweilige Position sowohl der Nachverfolgungseinrichtung als auch des Kontrollmarkers relativ zueinander durch deren Anordnung an dem Roboterarm beispielsweise vor einem Einsatz des Systems ermittelt werden kann, beispielsweise aus oder anhand einer Spezifikation des Systems oder durch entsprechende unabhängige Messung und/oder Kalibrierung, und während eines Einsatzes des robotischen Systems bei einer Bewegung des Roboterarms konstant bleibt. Das System ist weiterhin dazu eingerichtet, bei einer erkannten Differenz zwischen der gemessenen und der realen, vorgegebenen räumlichen Lagebeziehung in Abhängigkeit von der Differenz ein entsprechendes Signal zu erzeugen, insbesondere als Steuersignal auszugeben. Die erkannte Differenz kann also ein Ergebnis des Vergleichens der gemessenen und der vorgegebenen räumlichen Lagebeziehungen sein.

Das vorliegende System stellt vorteilhaft also ein integriertes System dar, bei dem die Nachverfolgungseinrichtung, die einem wesentlichen Teil herkömmlicher eigenständiger Navigationssysteme entsprechen kann, durch die Anordnung an dem Roboterarm mit dem Roboter integriert ist, also ein gemeinsames Gerät oder System bilden kann. Damit besteht vorteilhaft keine Abhängigkeit von einem zusätzlichen, separaten, externen Navigationssystem. Hierdurch können Inkompatibilitäten besonders einfach vermieden werden und/oder ein zusätzlicher Entwicklungs- oder Anpassungsaufwand, beispielsweise für eine auf das jeweilige System ausgelegte, individuelle Einzelfalllösung entfallen.

Dass die Nachverfolgungseinrichtung an dem Roboterarm befestigt ist bedeutet oder kann bedeuten, dass sich im Betrieb, also beispielsweise bei einem Einsatz oder bei einer Bewegung, des Roboters der Roboterarm und die Nachverfolgungseinrichtung gemeinsam als starres System bewegen oder bewegen können. Dadurch kann vorteilhaft ein separates Nachführen der Nachverfolgungseinrichtung entfallen, wie es bei einer Verwendung eines als eigenständiges separates Gerät ausgebildeten Navigationssystems herkömmlicherweise notwendig sein kann. Ebenso kann ein verbesserter Arbeitsfluss (Workflow) erreicht werden, da beispielsweise eine Wahrscheinlichkeit für Sichtverdeckungen zwischen der Nachverfolgungseinrichtung und dem Marker reduziert werden kann, da der Roboterarm ohnehin stets in Richtung des Patienten ausgerichtet ist und somit also die Problematik eliminiert werden kann, dass der Roboterarm zwischen den an dem Patienten angeordneten Marker und eine Nachverfolgungseinrichtung eines separaten Navigationssystems gerät.

Der Roboter kann beispielsweise ein medizinischer Roboter oder ein Industrieroboter mit wenigstens sechs, beispielsweise sechs bis zehn, Achsen oder Freiheitsgraden sein. Derartige Roboter weisen entsprechende Steuerungen und/oder Sensoren auf, welche eine jeweils aktuelle Position und Orientierung, also Pose, des Roboters überwachen und entsprechende Positionsdaten bereitstellen können. Ebenso kann das erfindungsgemäße System beispielsweise ein C-Bogen-Gerät umfassen. Der C-Bogen kann dabei beweglich sein, so dass er also ein Roboter oder Roboterarm im Sinne der vorliegenden Erfindung sein kann. Es kann in diesem Fall also beispielsweise möglich sein, die Nachverfolgungseinrichtung an dem C-Bogen anzuordnen. Der Kontrollmarker und/oder ein weiterer Marker, etwa ein Hilfsmarker, können beispielsweise in bekannter räumlicher Lagebeziehung oder Relation zu dem C-Bogen bzw. zu der Nachverfolgungseinrichtung an dem C-Bogen oder an einem Patientenlager angeordnet oder befestigt sein.

Da also die Positionsdaten bei der Verwendung des Roboters ohnehin vorliegen, kann aufgrund der bekannten Lagebeziehung zwischen der Nachverfolgungseinrichtung und dem Roboterarm vorteilhaft eine separate Sensorik oder ein separates Überwachen der Position und Orientierung der Nachverfolgungseinrichtung entfallen.

Dass die konstante, vorgegebene räumliche Lagebeziehung des Kontrollmarkers und/oder der Nachverfolgungseinrichtung relativ zu dem Roboterarm bei einem Betrieb des Roboters vorliegt oder gegeben ist, bedeutet oder schließt zumindest nicht aus, dass beispielsweise bei einem Stillstand des Roboters - und damit auch des Roboterarms - der Kontrollmarker und/oder die Nachverfolgungseinrichtung in ihrer jeweiligen Lage und/oder Orientierung verändert oder verstellt werden können. Die jeweilige Befestigung an dem Roboterarm ist also bevorzugt reversibel und zerstörungsfrei lösbar.

Ebenso wie der Marker kann auch der Kontrollmarker auf verschiedene Arten und Weisen ausgebildet sein, insbesondere abhängig von der oder abgestimmt auf die Art oder Ausgestaltung der Nachverfolgungseinrichtung. Ist die Nachverfolgungseinrichtung für ein optisches Nachverfolgen, also beispielsweise als Kamera, ausgebildet, so können der Marker und der Kontrollmarker beispielsweise als reflektive Metall- oder Kunststoffkugeln oder -halbkugeln ausgebildet sein. Ein optisches Nachverfolgen muss dabei nicht auf eine Verwendung von sichtbarem Licht beschränkt sein, sondern kann beispielsweise ebenso gut Infrarotlicht oder andere Frequenzen oder Wellenlängen verwenden. Ist die Nachverfolgungseinrichtung für ein elektromagnetisches Nachverfolgen (EMT, engl. "electromagnetic tracking) ausgebildet, so können der Marker und der Kontrollmarker beispielsweise wenigstens eine, bevorzugt mehrere, Empfängerspulen oder elektromagnetische Sensoren aufweisen. Hierbei kann die Nachverfolgungseinrichtung beispielsweise eine Anordnung von mehreren, beispielsweise von acht, Sendespulen aufweisen. Diese Sendespulen können dabei in einer bekannten räumlichen Lagebeziehung zueinander angeordnet sein, um unabhängig von einer konkreten Orientierung ein zuverlässiges Nachverfolgen zu ermöglichen. Die einzelnen Sendespulen können dabei beispielsweise in einer vorgegebenen Sequenz aktiviert werden, also in einer bestimmten Reihenfolge ein jeweiliges Nachverfolgungssignal aussenden. Dies ermöglicht es zusammen mit der bekannten Anordnung der Sendespulen aus einem an dem Marker beziehungsweise an dem Kontrollmarker empfangenen oder gemessenen Signal die räumliche Lagebeziehung zwischen der Nachverfolgungseinrichtung und dem Marker beziehungsweise dem Kontrollmarker zu ermitteln und damit also die Position und Orientierung des Markers beziehungsweise des Kontrollmarkers zu bestimmen.

In jedem Fall können die Nachverfolgungseinrichtung einerseits und der Marker beziehungsweise Kontrollmarker andererseits derart aufeinander abgestimmt oder aneinander angepasst sein, dass durch deren Zusammenwirken das Nachverfolgen ermöglicht wird. Um einen Aufwand zu minimieren und eine Überprüfbarkeit oder Zuverlässigkeitskontrolle des Nachverfolgens, also der Positionsbestimmung des Markers und damit des Zielobjekts, zu ermöglichen, können der Marker und der Kontrollmarker bevorzugt von gleicher Art sein. Bevorzugt sind die Nachverfolgungseinrichtung und der Marker beziehungsweise der Kontrollmarker und/oder die Datenverarbeitungseinrichtung, also das System insgesamt, dazu eingerichtet, die jeweilige Position und Lage in drei räumlichen Positionskoordinaten und drei räumlichen Winkeln zu bestimmen (6D Tracking). So kann vorteilhaft nicht nur die Position des Zielobjekts, sondern auch dessen Orientierung, also insgesamt die jeweilige Pose bestimmt werden.

Die Datenverarbeitungseinrichtung kann eine Prozessoreinrichtung, beispielsweise einen Mikrochip oder Mikrocontroller, sowie einen Datenspeicher umfassen. In diesem Datenspeicher können beispielsweise die von der Nachverfolgungseinrichtung und/oder dem Marker und/oder dem Kontrollmarker bereitgestellten Daten zwischengespeichert werden. Ebenso kann in dem Datenspeicher Programmcode abgelegt sein, der Verfahrensschritte für die erfindungsgemäße Nachverfolgungseinrichtung repräsentiert oder kodiert. Die Datenverarbeitungseinrichtung und somit das System können also dazu eingerichtet sein, den Programmcode zum Durchführen des Verfahrens mittels der Prozessoreinrichtung auszuführen. Die Datenverarbeitungseinrichtung kann zentralisiert, also monolithisch beispielsweise in oder an dem Roboter oder in einem separaten Gehäuse angeordnet sein. Sie kann beispielsweise über eine Datenverbindung oder eine Datenleitung mit dem Roboter verbunden sein. Ebenso kann die Datenverarbeitungseinrichtung jedoch verteilt angeordnet, also aus an verschiedenen Stellen angeordneten Komponenten zusammengesetzt sein. Als die Datenverarbeitungseinrichtung oder als Teil davon kann ebenso beispielsweise ein Rechner oder eine Steuereinrichtung dienen, welche beispielsweise gleichzeitig auch für andere Aufgaben verwendbar sein kann. Die Datenverarbeitungseinrichtung kann bevorzugt mit einer Anzeige- oder Visualisierungseinrichtung gekoppelt sein, um beispielsweise die nachverfolgte Position des Zielobjekts darzustellen. Ebenso kann hier beispielsweise eine aktuelle Zuverlässigkeit oder Güte des Nachverfolgens oder der ermittelten Position und/oder Orientierung des Zielobjekts visualisiert werden.

Es ist ein besonderer Vorteil der vorliegenden Erfindung, dass aus einem Vergleichsergebnis des Vergleichen der vorgegebenen räumlichen Lagebeziehung mit der entsprechenden gemessenen räumlichen Lagebeziehung zwischen der Nachverfolgungseinrichtung und dem Kontrollmarker die Zuverlässigkeit oder Güte des Nachverfolgens, also der Positionsbestimmung des Zielobjekts beziehungsweise des Markers, ermöglicht wird. Aufgrund der konstanten vorgegebenen räumlichen Lagebeziehung ist bei einem bestimmungsgemäßen und störungsfreien Betrieb damit zu rechnen, dass die Position des Kontrollmarkers relativ zu der Nachverfolgungseinrichtung, also beispielsweise deren Abstand zueinander, ein konstantes Signal oder Messergebnis liefert. Wird also im Betrieb eine Abweichung oder Differenz zu diesem erwarteten konstanten Signal oder Messergebnis gemessen oder festgestellt, so ist dies ein unmittelbares und zuverlässiges Indiz dafür, dass eine Störung vorliegt. In einem solchen Fall muss dann damit gerechnet werden, dass auch die gemessene Position des Zielobjekts von dessen tatsächlicher Position entsprechend abweichen kann. Gerade bei dem elektromagnetischen Nachverfolgen können Störungen oder Ungenauigkeiten auf nicht offensichtliche oder unmittelbar erkennbare Weise verursacht werden, beispielsweise durch metallische Gegenstände im Bereich des Markers oder durch von anderen Geräten ausgehende elektromagnetische Störfelder.

Die vorliegende Erfindung kann also vorteilhaft ohne zusätzliches Gerät und mit minimalem Platzbedarf die Zuverlässigkeit des Nachverfolgens des Zielobjekts angeben und somit zu einem verbesserten Behandlungsergebnis beitragen. Da für das Messen der räumlichen Lagebeziehung zwischen der Nachverfolgungseinrichtung und dem Kontrollmarker, also für das Nachverfolgen des Kontrollmarkers, dieselbe Nachverfolgungseinrichtung wie zum Nachverfolgen des Markers oder des Zielobjekts verwendet wird, können so vorteilhaft auch unmittelbar Funktionsstörungen oder störende Beeinflussungen der Nachverfolgungseinrichtung erkannt werden. Insgesamt wird in der vorliegenden Erfindung vorteilhaft also mit der gleichzeitigen Integration von Nachverfolgungseinrichtung und redundantem Kontrollmarker in oder an dem System eine Güteprüfung oder Zuverlässigkeitskontrolle implementiert, die etwaige Störungen der Nachverfolgungseinrichtung, des Nachverfolgens oder allgemein in einer Umgebung des Systems quantitativ erfassen und angeben oder rückmelden kann.

Besonders bevorzugt kann vorgesehen sein, dass sich bei einem Betrieb des erfindungsgemäßen System, also etwa in einer Arbeitsposition, der Kontrollmarker in räumlicher Nähe, bevorzugt in einer Entfernung von weniger als 1 m, besonders bevorzugt in einer Entfernung von weniger als 50 cm, zu dem Marker an dem Zielobjekt befindet. Dies kann beispielsweise dadurch erreicht werden, dass der Kontrollmarker in einem vorderen, also dem Zielobjekt zugewandten Endbereich, bevorzugt an einem an dem System gehaltenen oder als Teil des Systems vorgesehenen Werkzeugs angeordnet ist. So können vorteilhaft auf den oder im Bereich des Markers wirkende Störeinflüsse besonders zuverlässig erkannt werden, insbesondere auch dann, wenn diese eine entsprechende kurze räumliche Reichweite haben.

Im Folgenden ist das erfindungsgemäße System ohne Einschränkung der Anschaulichkeit halber auch als robotisches System bezeichnet.

In weiterer vorteilhafter Ausgestaltung der vorliegenden Erfindung kann die Nachverfolgungseinrichtung an einem Roboterflansch des Roboterarms oder an einem Fuß des Roboterarms, also einem Roboterfuß des Roboters, befestigt sein. Der Roboterfuß kann ein von dem Zielobjekt abgewandtes Ende des Roboterarms bilden und beispielsweise an einem Boden eines jeweiligen Einsatzraumes des robotischen Systems festgelegt oder montiert sein. Der Roboterflansch kann hingegen an einem entgegengesetzten, dem Zielobjekt zugewandten Ende des Roboterarms angeordnet sein. An dem Roboterflansch kann beispielsweise ein für eine Interaktion mit dem Zielobjekt vorgesehenes Werkzeug (Tool) befestigt werden oder sein. Ebenso können der Roboterflansch oder das Werkzeug ein Ende des Roboterarms bilden. Das Befestigen der Nachverfolgungseinrichtung an dem Roboterflansch bringt den Vorteil mit sich, dass die Nachverfolgungseinrichtung bei einem Einsatz des robotischen Systems automatisch auf das Zielobjekt ausgerichtet ist und besonders nahe an das Zielobjekt und damit an den Marker gebracht wird oder werden kann. Die damit einhergehende geringere Entfernung zwischen der Nachverfolgungseinrichtung und dem Marker kann vorteilhaft ein genaueres Nachverfolgen des Zielobjekts ermöglichen. Insbesondere das elektromagnetische Nachverfolgen weist eine begrenzte effektive Reichweite auf. Aber auch bei der Verwendung von optischem Tracking kann durch das Befestigen oder Anordnen der Nachverfolgungseinrichtung an dem Roboterflansch aufgrund der größeren Nähe zu dem Zielobjekt eine Wahrscheinlichkeit dafür minimiert werden, dass störende Objekte in die Sichtlinie zwischen der Nachverfolgungseinrichtung und dem Marker gelangen. Das Befestigen oder Anordnen der Nachverfolgungseinrichtung an dem Roboterfuß kann hingegen den Vorteil haben, in der unmittelbaren Nähe oder Umgebung des Zielobjekts keinen Raum zu belegen, also beispielsweise für einen behandelnden Arzt mehr Bewegungsfreiraum zu bieten.

In einer vorteilhaften Weiterbildung der vorliegenden Erfindung weist die Nachverfolgungseinrichtung einen elektromagnetischen Feldgenerator (EM-Feldgenerator), also beispielsweise eine oder mehrere Sendespulen, auf. Der Kontrollmarker - und bevorzugt auch der Marker - weist dann einen auf den EM-Feldgenerator abgestimmten Sensor, insbesondere wenigstens eine Empfängerspule, auf. In dieser Weiterbildung der Erfindung wird also ein elektromagnetisches Tracking zum Nachverfolgen der Position des Zielobjekts und zum Messen der räumlichen Lagebeziehung zwischen der Nachverfolgungseinrichtung und dem Kontrollmarker verwendet. Das elektromagnetische Tracking kann vorteilhaft weniger störungsanfällig gegenüber in die direkte Sichtlinie zwischen der Nachverfolgungseinrichtung und dem Kontrollmarker oder dem Marker an dem Zielobjekt gelangenden Objekten sein als ein optisches Tracking.

In einer vorteilhaften Weiterbildung der vorliegenden Erfindung weist die Nachverfolgungseinrichtung eine Kamera zum optischen Erfassen des Markers und des Kontrollmarkers auf. In dieser Weiterbildung der Erfindung wird also optisches Tracking zum Nachverfolgen der Position des Zielobjekts und zum Messen der räumlichen Lagebeziehung zwischen der Nachverfolgungseinrichtung und dem Kontrollmarker verwendet. Hierbei können der Kontrollmarker und/oder der Marker an dem Zielobjekt mehrere optisch von der Nachverfolgungseinrichtung erfassbare Merkmale oder Features aufweisen, welche ein Bestimmen der jeweiligen Orientierung im Raum ermöglichen. Ebenso können der Kontrollmarker und/oder der Marker dabei beispielsweise aus einem jeweiligen Ensemble mehrerer Teilmarker bestehen, welche in einer bekannten festen räumlichen Lagebeziehung zueinander angeordnet sind. Das optische Tracking kann vorteilhaft über größere räumliche Entfernungen hinweg ein zuverlässiges Nachverfolgen beziehungsweise Messen der jeweiligen Positionen oder räumlichen Lagebeziehungen ermöglichen, als dies beispielsweise mit einem elektromagnetischen Tracking möglich ist. Ebenso kann gegenüber dem elektromagnetischen Tracking durch die Verwendung des optischen Trackings eine Störanfälligkeit gegenüber elektromagnetischen Einflüssen und/oder beispielsweise in die Nähe des Kontrollmarkers und/oder des Markers gelangenden metallischen Objekten reduziert werden.

In einer vorteilhaften Weiterbildung der vorliegenden Erfindung sind die Nachverfolgungseinrichtung und/oder der Kontrollmarker zum Vorgeben ihrer räumlichen Lagebeziehung zueinander beweglich aber fixierbar an dem Roboterarm befestigt. Mit anderen Worten kann dann also die Lage und/oder Orientierung der Nachverfolgungseinrichtung und/oder des Kontrollmarkers relativ zu dem Roboterarm ver- oder eingestellt und anschließend in einer neuen Position und Orientierung fixiert werden. Dazu können die Nachverfolgungseinrichtung und/oder der Kontrollmarker beispielsweise mittels eines arretierbaren Schwenk- oder Gelenkarms an dem Roboterarm befestigt sein. Hierdurch kann vorteilhaft bedarfsgerecht sichergestellt werden, dass sich sowohl der Kontrollmarker als auch der Marker an dem Zielobjekt in einem Sicht- oder Erfassungsbereich der Nachverfolgungseinrichtung befinden, also angeordnet werden können. Wird die Nachverfolgungseinrichtung oder wird der Kontrollmarker derart verstellt oder neu ausgerichtet während sich der Roboterarm und das Zielobjekt in Ruhe befinden, so kann vorteilhaft eine automatische Re- oder Neu-Registrierung durchgeführt werden. Hierdurch kann also die neue räumliche Lagebeziehung zwischen der Nachverfolgungseinrichtung und dem Kontrollmarker, also die jeweilige neue Position und/oder Orientierung, automatisch ermittelt und für ein nach dem jeweiligen Fixieren durchgeführtes weiteres Nachverfolgen der Position des Zielobjekts und beim Messen der dann neuen vorgegebenen räumlichen Lagebeziehung zwischen der Nachverfolgungseinrichtung und dem Kontrollmarker berücksichtigt werden. Hier können beispielsweise die von der Nachverfolgungseinrichtung und/oder dem Kontrollmarker und/oder dem Marker gelieferten Daten mit Bilddaten eines bildgebenden Systems, beispielsweise einem Röntgengerät, registriert werden. Die vorliegende Erfindung bietet gegenüber herkömmlichen Systemen also den Vorteil einer verbesserten Flexibilität und einer vereinfachten Handhabbarkeit, da aufgrund der bekannten vorgegebenen räumlicher Lagebeziehung automatisch genügend Informationen zur Verfügung stehen, um bei einer Neuausrichtung eines Elements, also beispielsweise der Nachverfolgungseinrichtung oder des Kontrollmarkers, die Neu-Registrierung automatisch durchführen zu können.

In einer vorteilhaften Weiterbildung der vorliegenden Erfindung weist das robotische System zusätzlich ein externes, insbesondere weder an dem Roboter noch an dem Zielobjekt befestigtes, Ensemble von Hilfsmarkern auf. Das robotische System ist dabei dazu eingerichtet, beim Nachverfolgen der Position des Zielobjekts eine räumliche Lagebeziehung zwischen der Nachverfolgungseinrichtung und dem Ensemble von Hilfsmarkern und/oder eine räumliche Lagebeziehung der Hilfsmarker untereinander, also relativ zueinander, zu messen und das Nachverfolgen der Position des Zielobjekts anhand eines entsprechenden Messergebnisses zu plausibilisieren. Die Hilfsmarker können beispielsweise ebenso ausgebildet sein wie der Kontrollmarker oder wie der Marker an dem Zielobjekt.

Das Plausibilisieren des Nachverfolgens der Position des Zielobjekts, das heißt also der ermittelten Position und Orientierung des Zielobjekts, kann beispielsweise umfassen oder bedeuten, die gemessene räumliche Lagebeziehung der Hilfsmarker oder zu den Hilfsmarkern mit dem entsprechenden bei einem störungsfreien Betrieb erwarteten Messwert oder Messergebnis zu vergleichen. Ebenso kann für das Plausibilisieren überprüft werden, ob zwischen dem gemessenen und dem erwarteten Wert oder Signal eine Abweichung oder Differenz besteht, die größer ist als ein vorgegebener Schwellenwert. Dieser Schwellenwert kann beispielsweise als Störschwellenwert bezeichnet werden.

Die Hilfsmarker können untereinander, also relativ zueinander bevorzugt in einer bekannten festen Lagebeziehung angeordnet sein, welche insbesondere bevorzugt aus jeder Betrachtungsrichtung oder Perspektive ein eindeutiges Bestimmen der Orientierung des Ensembles von Hilfsmarkern im Raum ermöglicht. Die Hilfsmarker können unterschiedlich positioniert sein oder werden, beispielsweise fest relativ zu dem Roboter oder relativ zu dem Zielobjekt und/oder beispielsweise fest, also lagestabil, in einem externen Koordinatensystem, welches beispielsweise durch Wände, Boden und Decke eines Einsatzraumes, in dem sich das robotische System befindet, definiert sein kann.

Durch die Verwendung der zusätzlichen Hilfsmarker und deren bekannte räumliche Position und Lage kann vorteilhaft eine verbesserte Flexibilität und Güte des Nachverfolgens des Zielobjekts und des Bestimmens der Zuverlässigkeit dieses Nachverfolgens erreicht werden. Ähnlich wie im Zusammenhang mit dem Kontrollmarker beschrieben, ist bei einem störungsfreien Betrieb des robotischen Systems ein im Vorhinein bekanntes Signal oder Messergebnis für die räumliche Lagebeziehung zwischen der Nachverfolgungseinrichtung und den Hilfsmarkern und/oder für die räumliche Lagebeziehung der Hilfsmarker untereinander zu erwarten. Dies kann in ersterem Fall ein sich in einer bestimmten erwarteten Art und Weise veränderndes Signal oder in letzterem Fall ein bestimmtes konstantes Signal oder Messergebnis von konstanter Größe sein. Wird im Betrieb dann eine Abweichung hiervon festgestellt, so kann auf eine Störung oder eine eingeschränkte Genauigkeit oder Zuverlässigkeit bei dem Nachverfolgen der Position des Zielobjekts geschlossen werden. Auch wenn anhand der Hilfsmarker eine derartige Abweichung oder Differenz durch einen entsprechenden Vergleich festgestellt, also eine Störung erkannt wird, kann von der Datenverarbeitungseinrichtung automatisch das oder ein entsprechendes Signal oder Steuersignal erzeugt beziehungsweise ausgegeben werden.

Durch die Verwendung des Ensembles, also der mehreren Hilfsmarker, kann vorteilhaft sichergestellt werden, dass über einen besonders großen Schwenk- oder Bewegungsbereich des Roboterarms stets wenigstens einer oder einige der Hilfsmarker im Sichtfeld oder Erfassungsbereich der Nachverfolgungseinrichtung ist beziehungsweise sind. Bei einer Bewegung des Roboterarms können also in einem zeitlichen Verlauf einzelne der Hilfsmarker in den Erfassungsbereich der Nachverfolgungseinrichtung eintreten und/oder diesen verlassen. Auch dieser zeitliche Verlauf des Ein- und/oder Auftretens der Hilfsmarker in den beziehungsweise aus dem Erfassungsbereich der Nachverfolgungseinrichtung - und/oder die entsprechenden Ein- und/oder Austrittszeitpunkte - können beispielsweise durch die Datenverarbeitungseinrichtung automatisch zum Überprüfen oder Bewerten der Nachverfolgung des Zielobjekts, also mit anderen Worten zum Erkennen von Störungen, ausgewertet werden. Es kann dabei besonders vorteilhaft sein, wenn die einzelnen Hilfsmarker des Ensembles jeweilige Identifizierungsmerkmale aufweisen, anhand derer sie durch die Nachverfolgungseinrichtung und/oder durch die Datenverarbeitungseinrichtung eindeutig und individuell identifizierbar sind. Das Ensemble von Hilfsmarkern kann dabei mehrere räumlich verteilt angeordnete Gruppen von Hilfsmarkern umfassen. In jeder Gruppe von Hilfsmarkern können die jeweiligen Hilfsmarker bevorzugt so relativ zueinander angeordnet sein, dass aus jeder Betrachtungsrichtung ein eindeutiges Bestimmen der räumlichen Orientierung dieser Gruppe von Hilfsmarkern ermöglicht wird.

Zusätzlich oder alternativ zu den Hilfsmarkern können in entsprechender Art und Weise beispielsweise eine oder mehrere zusätzliche Nachverfolgungseinrichtungen vorgesehen sein. Hiermit kann beispielsweise der Kontrollmarker aus einer bekannten alternativen Perspektive nachverfolgt werden. Dies schafft also ebenfalls eine zusätzliche Redundanz, die die Zuverlässigkeit des Nachverfolgens des Zielobjekts und/oder die Zuverlässigkeit der Erkennung von Störungen ebenso wie die Flexibilität beim Anordnen und/oder Ausrichten des Roboterarms und/oder der an dem Roboterarm angeordneten Nachverfolgungseinrichtung verbessern kann.

Die erfindungsgemäße Nachverfolgungseinrichtung dient zum Nachverfolgen der Position, insbesondere der Position und Orientierung eines Zielobjekts, insbesondere eines Patienten, mittels eines, insbesondere robotischen, Systems. Dabei wird die Position des Zielobjekts mittels einer an einem beweglichen Roboterarm eines Roboters befestigten Nachverfolgungseinrichtung und eines an dem Zielobjekt angeordneten Markers nachverfolgt. In einem weiteren Verfahrensschritt wird bei dem Nachverfolgen eine räumliche Lagebeziehung zwischen der Nachverfolgungseinrichtung und wenigstens einem in einer vorgegebenen, bei einer Bewegung bei einem Betrieb des Roboters konstanten bekannten räumlichen Lagebeziehung zu der Nachverfolgungseinrichtung angeordneten Kontrollmarker mittels der Nachverfolgungseinrichtung gemessen. Der Kontrollmarker kann ebenfalls an dem Roboterarm befestigt oder angeordnet sein. In einem weiteren Verfahrensschritt wird diese gemessene räumliche Lagebeziehung mit der entsprechenden realen, vorgegebenen räumlichen Lagebeziehung verglichen. Wenn eine Differenz zwischen der gemessenen und der realen, vorgegebenen räumlichen Lagebeziehung erkannt wird, wird in einem weiteren Verfahrensschritt von dem System in Abhängigkeit von der erkannten Differenz ein entsprechendes Signal erzeugt. Das Verfahren kann also beispielsweise mittels eines erfindungsgemäßen robotischen Systems durchgeführt werden.

In vorteilhafter Weiterbildung des Verfahrens wird das Signal als Steuersignal ausgegeben.

In vorteilhafter Weiterbildung des Verfahrens wird auf das Signal beziehungsweise das Steuersignal hin eine Warnung über die erkannte Differenz ausgegeben. Das Signal kann also ein Steuersignal für eine Warneinrichtung sein und entsprechend an die Warneinrichtung ausgegeben oder übermittelt werden, um diese zum Ausgeben der - beispielsweise akustischen und/oder visuellen - Warnung zu veranlassen. Eine Störung oder ein Verlust oder eine Verringerung einer Genauigkeit oder Zuverlässigkeit bei dem Nachverfolgen der Position des Zielobjekts kann üblicherweise von einem jeweiligen Nutzer oder Anwender nicht ohne weiteres erkannt werden. Durch die Warnung kann der Nutzer, beispielsweise ein behandelnder Arzt, vorteilhaft hierauf aufmerksam gemacht werden. So kann vorteilhaft vermieden werden, dass der jeweilige Nutzer sich auch bei einem Vorliegen einer Störung unbesehen auf das robotische System beziehungsweise die Positionsnachverfolgung des Zielobjekts verlässt. Das Verfahren, insbesondere die Warnung können also beispielsweise dazu beitragen, Fehler oder Komplikationen bei einer Behandlung des Patienten zu vermeiden.

In vorteilhafter Weiterbildung des Verfahrens wird nach einer Veränderung der vorgegebenen räumlichen Lagebeziehung zwischen der Nachverfolgungseinrichtung und dem Kontrollmarker, bei der entweder die Nachverfolgungseinrichtung oder der Kontrollmarker verstellt wird, von dem System automatisch eine Registrierung oder Neu-Registrierung mit einem vorgegebenen Koordinatensystem oder entsprechenden Bilddaten, insbesondere eines bildgebenden Systems, durchgeführt. Die Registrierung wird dabei anhand des unverstellten Kontrollmarkers beziehungsweise anhand der unverstellten Nachverfolgungseinrichtung beziehungsweise entsprechender gemessener und/oder bekannter Positionsdaten vorgenommen. Die Veränderung der vorgegebenen räumlichen Lagebeziehung wird dabei während eines Stillstandes, insbesondere relativ zu dem Zielobjekt, des Roboterarms vorgenommen. Dadurch ist sichergestellt, dass nur ein Element oder eine Größe innerhalb des Gesamtsystems aus dem robotischen System und dem Zielobjekt verändert wird, wodurch die damit notwendig werdende Neu-Registrierung besonders einfach und aufwandsarm durchgeführt werden kann. Dieses Vorgehen ermöglicht es vorteilhaft also, flexibel auf situationsbedingte Gegebenheiten zu reagieren, um beispielsweise sicherzustellen, dass sich der Kontrollmarker und der an dem Zielobjekt angeordnete Marker weiterhin oder auch bei zukünftigen Bewegungen des Roboterarms in dem Blickfeld oder Erfassungsbereich der Nachverfolgungseinrichtung befinden.

In vorteilhafter Weiterbildung des Verfahrens wird aus der erkannten Differenz ein Korrekturwert bestimmt. Dieser Korrekturwert wird dann bei dem Nachverfolgen der Position des Zielobjekts automatisch berücksichtigt, um einen die Differenz verursachenden Störeinfluss zu kompensieren. So kann beispielsweise die reale Position und Lage des Zielobjekts mit verbesserter Genauigkeit und Zuverlässigkeit visualisiert oder angezeigt werden, insbesondere unter einer größeren Vielfalt von Bedingungen. Das Signal kann also beispielsweise ein Steuersignal für eine Anzeigeeinrichtung oder für eine Einrichtung, die die Visualisierung der Position und Lage des Zielobjekts erzeugt oder steuert, sein. Der Korrekturwert kann also beispielsweise als Offset gehandhabt oder interpretiert werden, sodass dann durch das Steuersignal ein entsprechendes Verschieben und/oder Rotieren der Visualisierung oder Darstellung des Zielobjekts oder dessen Position gemäß dem Korrekturwert veranlasst werden kann.

Das Signal kann also als Steuersignal an eine externe Einrichtung oder ein externes Gerät ausgegeben werden. Ebenso kann das Signal jedoch ein internes Signal sein, also an eine interne Funktionseinheit des robotischen Systems oder der Datenverarbeitungseinrichtung ausgesendet, also übermittelt werden. Letzteres kann beispielsweise dann der Fall sein, wenn die Einrichtung zum Visualisieren des Zielobjekts oder der räumlichen Lage des Zielobjekts Teil des robotischen Systems ist.

Die bisher und im Folgenden angegebenen Eigenschaften und Weiterbildungen des erfindungsgemäßen robotischen Systems und des Verfahrens für die Nachverfolgungseinrichtung sowie die entsprechenden Vorteile sind jeweils sinngemäß wechselseitig zwischen diesen Aspekten der vorliegenden Erfindung übertragbar. Dies gilt auch für zur Durchführung des Verfahrens verwendete oder verwendbare Bauteile und Einrichtungen. Es gehören also zu der Erfindung auch solche Weiterbildungen des erfindungsgemäßen robotischen Systems und des Verfahrens, welche Ausgestaltungen aufweisen, die hier nicht explizit in der jeweiligen Kombination beschrieben sind.

Weitere Merkmale, Einzelheiten und Vorteile der vorliegenden Erfindung ergeben sich aus der nachfolgenden Beschreibung bevorzugter Ausführungsbeispiele sowie anhand der Zeichnungen. Dabei zeigt:
- FIG 1: eine schematische Seitenansicht einer eines robotischen Systems mit einer Nachverfolgungseinrichtung bei einem Einsatz an einem Patienten;
- FIG 2: eine schematische Seitenansicht der Situation aus FIG 1, wobei die Nachverfolgungseinrichtung an anderer Stelle an dem robotischen System angeordnet ist; und
- FIG 3: einen beispielhaften schematischen Ablaufplan eines beispielhaften Verfahrens zum Nachverfolgen einer Position eines Zielobjekts.

Bei den im Folgenden erläuterten Ausführungsbeispielen handelt es sich um bevorzugte Ausführungsformen der Erfindung. Bei den Ausführungsbeispielen stellen die beschriebenen Komponenten der Ausführungsformen jeweils einzelne, unabhängig voneinander zu betrachtende Merkmale der Erfindung dar, welche die Erfindung jeweils auch unabhängig voneinander weiterbilden und damit auch einzeln oder in einer anderen als der gezeigten Kombination als Bestandteil der Erfindung anzusehen sind. Des Weiteren sind die beschriebenen Ausführungsformen auch durch weitere der bereits beschriebenen Merkmale der Erfindung ergänzbar.

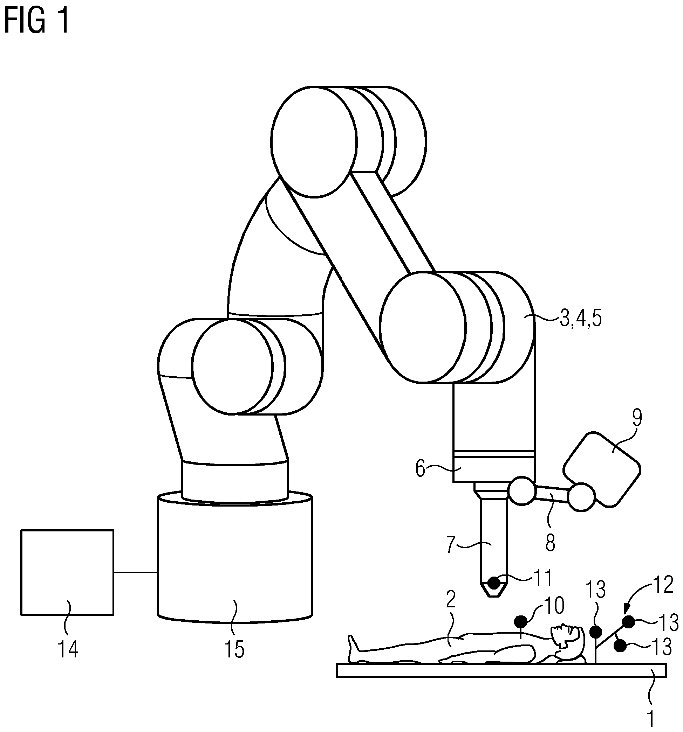

FIG 1 zeigt eine schematische Seitenansicht einer Behandlungssituation. Darin liegt auf einem Patientenlager 1 ein Patient 2, der mittels eines robotischen Systems 3 behandelt wird oder werden soll. Das robotische System 3 weist vorliegend einen Roboter 4 mit einem beweglichen, mehrachsigen Roboterarm 5 auf. An einem Ende des Roboterarms 5 befindet sich ein Roboterflansch 6, über den ein Werkzeug 7 an dem Roboterarm 5 befestigt ist. Weiterhin ist an dem Roboterflansch 6 - und damit zumindest mittelbar an dem Roboterarm 5 - über einen Gelenkarm 8 eine Nachverfolgungseinrichtung 9 befestigt.

Der Roboterflansch 6 und/oder das Werkzeug 7 können dabei aber ebenso Teil des Roboterarms 5 selbst sein. Die Nachverfolgungseinrichtung 9 kann einen EM-Feldgenerator und/oder eine, bevorzugt stereoskopische, Kamera aufweisen. Die Nachverfolgungseinrichtung 9 dient zum Nachverfolgen (Tracking) einer Position und Orientierung des Patienten 2.

Dazu ist vorliegend an dem Patienten 2 ein Marker 10 angeordnet. Die Nachverfolgungseinrichtung 9 ist so ausgerichtet, dass dich der Marker 10 in deren Erfassungsbereich befindet. Das robotische System 3 weist zusätzlich einen Kontrollmarker 11 auf, der sich vorliegend beispielhaft an einer Spitze des Werkzeugs 7 befindet. Ebenso kann der Kontrollmarker 11 jedoch unmittelbar an dem Roboterarm 5 oder beispielsweise an dem Roboterflansch 6 angeordnet sein. Ebenso kann der Kontrollmarker 11 statt der hier dargestellten unmittelbaren Anordnung beispielsweise ähnlich der Nachverfolgungseinrichtung 9 über einen Gelenkarm angebunden sein. Die Nachverfolgungseinrichtung 9 und der Kontrollmarker 11 befinden sich stets in einer bekannten, konstanten, vorgegebenen räumlichen Lagebeziehung zueinander und zu dem Roboterarm 5. Demgegenüber kann sich die räumliche Lagebeziehung zwischen der Nachverfolgungseinrichtung 9 und dem Marker 10 bei einer Bewegung des Roboterarms 5 selbst verständlich ändern. Es ist vorgesehen, dass sich auch der Kontrollmarker 11 in dem Erfassungsbereich der Nachverfolgungseinrichtung 9 befindet.

Zusätzlich ist hier ein Ensemble 12 von Hilfsmarkern 13, vorliegend beispielhaft an dem Patientenlager 1 in der Nähe des Patienten 2, vorgesehen. Die Hilfsmarker 13 befinden sich damit vorliegend in einer festen räumlichen Lagebeziehung zu dem Marker 10 und damit zu dem Patienten 2. Außerdem befinden sich die Hilfsmarker 13 in einer bekannten, festen räumlichen Lagebeziehung relativ zueinander. Die Hilfsmarker 13 können ebenso wie der Marker 10 und der Kontrollmarker 11 von der Nachverfolgungseinrichtung erfasst werden. Aufgrund der Anordnung der Hilfsmarker 13 kann dabei die Lage und Orientierung des Ensembles 12 aus jeder Perspektive der Nachverfolgungseinrichtung 9 eindeutig bestimmt werden. Das Ensemble 12 kann sich während der Behandlung, also während eines Einsatzes des robotischen Systems 3 permanent oder nur zeitweise ganz oder teilweise in dem Erfassungsbereich der Nachverfolgungseinrichtung 9 befinden.

Von der Nachverfolgungseinrichtung 9, dem Marker 10, dem Kontrollmarker 11 und/oder den Hilfsmarkern 13 erzeugte Signale oder Daten können von einer Datenverarbeitungseinrichtung 14 des robotischen Systems 3 verarbeitet werden. Aus diesen Signalen oder Daten kann die Datenverarbeitungseinrichtung 14, beispielsweise in einem vorgegebenen Koordinatensystem, jeweilige absolute und/oder relative Positionen und Orientierungen der Nachverfolgungseinrichtung 9, des Markers 10 und damit des Patienten 2, des Kontrollmarkers 11 und des Ensembles 12 berechnen. Somit kann also die Position beziehungsweise räumliche Lage des Roboterarms 5, insbesondere des Werkzeugs 7, relativ zu dem Patienten 2 bestimmt und nachverfolgt werden.

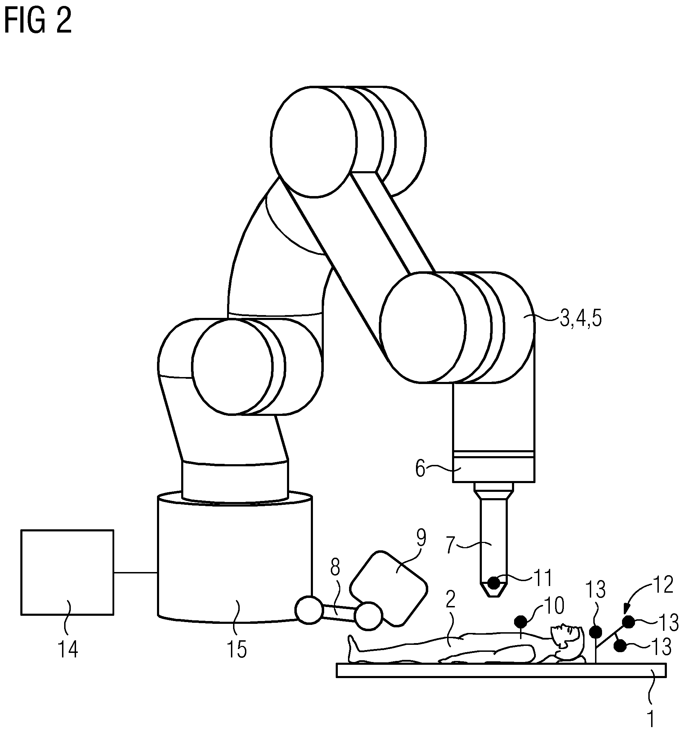

FIG 2 zeigt die gleiche schematische Seitenansicht wie FIG 1 mit dem Unterschied, dass hier die Nachverfolgungseinrichtung 9 nicht an dem Roboterflansch 6, sondern an einem Fuß 15 des Roboters 4 befestigt ist. Es kann sich hier um eine alternative Ausführungsform des robotischen Systems 3 handeln. Ebenso kann jedoch die Nachverfolgungseinrichtung 9 lediglich an demselben robotischen System 3 ummontiert sein, um beispielsweise eine räumliche Einschränkung, etwa durch ein hier nicht dargestelltes weiteres Gerät, zu umgehen.

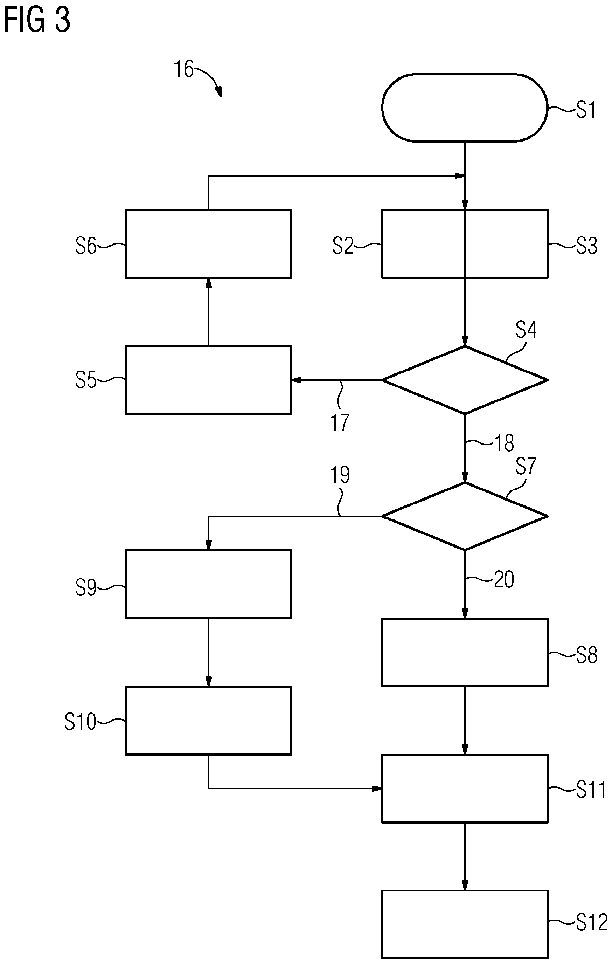

FIG 3 zeigt einen beispielhaften schematischen Ablaufplan 16 eines beispielhaften Verfahrens zum Nachverfolgen der Position und Orientierung eines Zielobjekts, beispielsweise des Patienten 2, insbesondere relativ zu dem robotischen System 3.

Im Folgenden wird das Verfahren anhand von FIG 3 und unter Bezugnahme auf FIG 1 und FIG 2 erläutert. Das Verfahren kann mit einem Verfahrensschritte S1 beginnen. Hier können beispielsweise die Nachverfolgungseinrichtung 9 und das Ensemble 12 vorgegeben positioniert werden. Ebenso können die räumlichen Lagebeziehungen zwischen der Nachverfolgungseinrichtung 9 und dem Kontrollmarker 11 sowie zwischen dem Ensemble 12 und dem Marker 10 und/oder beispielsweise zwischen dem Ensemble 12 und dem Fuß 15 vorgegeben und/oder ermittelt, bestimmt werden, damit sie im folgenden Verfahren als bekannte Größen zur Verfügung stehen. Ebenso können die notwendigen Registrierungen vorgenommen werden, beispielsweise des Markers 10 mit dem Patienten 2 beziehungsweise mit Bilddaten oder einem Koordinatensystem eines hier nicht dargestellten bildgebenden Systems, beispielsweise eines Röntgensystems. Hierfür können entsprechende Röntgenbilder aufgenommen und die Position des Markers 10 in den Röntgenbildern ausgewertet werden. Zusätzlich oder alternativ kann beispielsweise ein hier nicht dargestellter Markierungsrahmen (Markerframe) in vorgegebener Weise an dem oder um den Patienten 2 oder an dem bildgebenden System 2 angeordnet werden. Schließlich kann im Verfahrensschritt S1 das robotische System 3 in Betrieb genommen werden.

In einem Verfahrensschritts S2 kann die vorgegebene und somit an sich bekannte räumliche Lagebeziehung zwischen der Nachverfolgungseinrichtung 9 und dem Kontrollmarker 11 und gegebenenfalls dem Ensemble 12 mittels des robotischen Systems 3, also von diesem selbst, gemessen werden. Beispielsweise parallel dazu kann in einem Verfahrensschritts S3 mit der Positionsbestimmung und dem Nachverfolgen der Position des Markers 10, also des Patienten 2 begonnen werden.

In einem Verfahrensschritt S4 kann beispielsweise durch einen entsprechenden Vergleich eine Differenz zwischen der im Verfahrensschritts S2 gemessenen räumlichen Lagebeziehung oder Lagebeziehungen und dem vorgegebenen, also in einem störungsfreien Betrieb auch als Messergebnis erwarteten jeweiligen Wert, berechnet werden. Ist die berechnete Differenz kleiner als ein vorgegebener Schwellenwert, so arbeiten das robotische System 3 und das Nachverfolgen der Position des Patienten 2 korrekt, also mit ausreichender Zuverlässigkeit und/oder Genauigkeit. Die Behandlung des Patienten 2, also der Einsatz des robotischen Systems kann dann begonnen oder fortgesetzt werden. Das Messen oder Überwachen der räumlichen Lagebeziehung zwischen der Nachverfolgungseinrichtung 9 und dem Kontrollmarker 11 dient also dazu, eine Integrität der Positionsmessung und -verfolgung des Markers 10 und somit des Patienten 2 sicherzustellen.

Während der Behandlung beziehungsweise während des Verfahrens kann dann beispielsweise einem Pfad 17 zu einem Verfahrensschritt S5 gefolgt werden. Hier kann beispielsweise die Nachverfolgungseinrichtung 9 in eine andere Stellung verstellt oder ummontiert werden, etwa wie dies in FIG 2 im Vergleich zu FIG 1 dargestellt ist. Die Nachverfolgungseinrichtung 9 wird dabei nach dem Verstellen oder Ummontieren in ihrer neuen Position und Orientierung fixiert und steht auch dann wieder in einer konstanten, vorgegebenen räumlichen Lagebeziehung zu dem Kontrollmarker 11. Die einstellbare Anordnung oder Fixierbarkeit der Nachverfolgungseinrichtung 9 und/oder des Kontrollmarkers 9 ermöglicht vorteilhaft das Beseitigen oder Umgehen von Line-of-Sight-Problemen, das heißt von Sichtverdeckungen oder Hindernissen, die das jeweilige Nachverfolgen oder Messen behindern würden. Im Gegensatz zu herkömmlichen Verfahren, bei denen ein separates Navigationssystem verwendet wird, ergibt sich vorliegend der Vorteil, dass das Verstellen der Nachverfolgungseinrichtung 9 und/oder des Kontrollmarkers 11 direkt an demjenigen Gerät - hier also direkt an dem robotischen System 3 oder dem Roboter 4 - vorgenommen werden kann, das ohnehin von dem Benutzer, beispielsweise dem behandelnden Arzt, bedient wird und sich damit stets in dessen Reichweite befindet. Dies ermöglicht vorteilhaft einen optimierten, besonders effizienten Arbeitsfluss (Workflow).

In einem Verfahrensschritt S6 kann dann eine automatische Neuregistrierung durchgeführt werden, um der neuen Relativposition der Nachverfolgungseinrichtung 9 Rechnung zu tragen. Anschließend kann das robotische System 3 wie zuvor weiterverwendet werden, was hier durch eine Schleife zurück zu den Verfahrensschritten S2 und S3 angedeutet ist. Die Verfahrensschritte S2 und S3 können also kontinuierlich während des Einsatzes des robotischen Systems 3 ausgeführt werden.

Wird im Verfahrensschritt S4 festgestellt, dass die Differenz größer als der vorgegebene Schwellenwert ist, so kann einem Pfad 18 zu einem Verfahrensschritts S7 gefolgt werden. Darin kann überprüft werden, ob die festgestellte Differenz kompensierbar ist, ob also ein Korrekturwert berechnet werden kann, welcher bei dem Nachverfolgen der Position und Lage des Patienten 2 zum Ausgleich einer die Differenz verursachenden Störung berücksichtigt werden kann - beispielsweise durch die Datenverarbeitungseinrichtung 14. Ist dies der Fall, so kann einem Pfad 19 folgend in einem Verfahrensschritt S9 der entsprechende Korrekturwert berechnet werden. Hierfür kann beispielsweise eine vorherige Kalibrierung notwendig sein, welche beispielsweise ebenfalls im Verfahrensschritt S1 durchgeführt werden kann.

In einem nachfolgenden Verfahrensschritt S10 kann dann basierend auf dem berechneten Korrekturwert ein Korrektur-Steuersignal erzeugt werden, beispielsweise für eine hier nicht dargestellte Visualisierungseinrichtung.

Ergibt sich im Verfahrensschritts S7, dass ein Kompensieren der Differenz nicht möglich ist, so kann das Verfahren einem Pfad 20 folgen. Dies kann beispielsweise der Fall sein, wenn die Differenz zu groß ist, also beispielsweise oberhalb eines vorgegebenen Korrektur-Schwellenwertes liegt, und/oder beispielsweise Schwankungen aufweist, etwa aufgrund von hochfrequenten Störeinflüssen.

Dem Pfad 20 folgend kann in einem Verfahrensschritt S8 dann ein alternatives, also von dem Korrektur-Steuersignal verschiedenes Steuersignal erzeugt werden.

Unabhängig davon, ob dem Pfad 19 oder dem Pfad 20 gefolgt wurde, kann in einem Verfahrensschritts S11 das jeweilige erzeugte Steuersignal ausgegeben werden. Ein jeweiliges Ausgabeziel, also eine Einrichtung oder Funktionseinheit, an die das jeweilige Steuersignal ausgegeben wird, kann davon abhängig sein oder abhängig davon ausgewählt werden oder bestimmt sein, welches Steuersignal erzeugt worden ist. Während also das im Verfahrensschritt S10 erzeugte Korrektur-Steuersignal beispielsweise an eine Visualisierungseinrichtung ausgegeben werden kann, kann das im Verfahrensschritt S8 erzeugte Steuersignal beispielsweise an eine Warneinrichtung zum Ausgeben einer Warnung übermittelt werden.

In einem Verfahrensschritts S12 kann dann die durch das jeweilige Steuersignal ausgelöste oder veranlasste Reaktion erfolgen. Dies kann also beispielsweise eine Korrektur der Visualisierung oder Darstellung der nachverfolgten Position des Patienten 2 und/oder das Ausgeben der Warnung sein. Es kann vorteilhaft vorgesehen sein, zusätzlich oder alternativ zu der Warnung beispielsweise den Roboter 3 oder den Roboterarm 5 zu stoppen, wenn die Differenz den vorgegebenen Schwellenwert überschreitet und nicht kompensierbar ist. Hierdurch kann vorteilhaft verhindert werden, dass der Roboterarm 5, insbesondere das Werkzeug 7, in für einen jeweiligen Benutzer nicht überprüfbarer Art und Weise bewegt wird.

Insgesamt ist vorliegend also ein robotisches System 3 mit integriertem Tracking beschrieben. Dabei ist unterschieden zwischen dem Marker 10, der an dem nachzuverfolgenden Zielobjekt angeordnet ist, und dem Kontrollmarker 11, der ausschließlich der Kontrolle oder Überprüfung dieser Nachverfolgung dient.

## Patentansprüche

1. System (3), aufweisend einen Roboter (4) mit einem beweglichen Roboterarm (5) und eine Nachverfolgungseinrichtung zum Nachverfolgen einer Position eines Zielobjekts (2), insbesondere eines Patienten (2), anhand eines an dem Zielobjekt (2) angeordneten Markers (10) durch Verarbeiten von von der Nachverfolgungseinrichtung (9) und/oder dem Marker (10) bereitgestellten Daten mittels einer Datenverarbeitungseinrichtung (14) des Systems (3),
wobei
- die Nachverfolgungseinrichtung (9) an dem Roboterarm (5) befestigt ist,
- das System (3) zusätzlich wenigstens einen Kontrollmarker (11) aufweist, und
- das System (3) dazu eingerichtet ist, die räumliche Lagebeziehung zwischen der Nachverfolgungseinrichtung (9) und dem Kontrollmarker (11) mittels der Nachverfolgungseinrichtung (9) zu messen,
**dadurch gekennzeichnet, dass**
- das System (3) dazu eingerichtet ist, die räumliche Lagebeziehung zwischen der Nachverfolgungseinrichtung (9) und dem Kontrollmarker (11) mittels der Nachverfolgungseinrichtung (9) bei dem Nachverfolgen der Position des Zielobjekts (2) zu messen
- der Kontrollmarker (11) in einer vorgegebenen bei einer Bewegung des Roboterarms (5) bei einem Betrieb des Roboters (4) konstanten räumlichen Lagebeziehung zu der Nachverfolgungseinrichtung (9) angeordnet ist, und
- das System (3) dazu eingerichtet ist,
- die gemessene räumliche Lagebeziehung mit der vorgegebenen räumlichen Lagebeziehung zu vergleichen und
- bei einer erkannten Differenz zwischen der gemessenen und der vorgegebenen räumlichen Lagebeziehung ein entsprechendes Signal zu erzeugen.

2. System (3) nach Anspruch 1, **dadurch gekennzeichnet, dass** der Kontrollmarker (11) ebenfalls an dem Roboterarm (5) befestigt ist.

3. System (3) nach einem der vorhergehenden Ansprüche, **dadurch gekennzeichnet, dass** die Nachverfolgungseinrichtung (9) an einem Roboterflansch (6) oder einem Fuß (15) des Roboterarms (5) befestigt ist.

4. System (3) nach einem der vorhergehenden Ansprüche, **dadurch gekennzeichnet, dass** die Nachverfolgungseinrichtung (9) einen EM-Feldgenerator aufweist und der Kontrollmarker (11) einen auf den EM-Feldgenerator abgestimmten Sensor, insbesondere wenigstens eine Empfängerspule, aufweist.

5. System (3) nach einem der vorhergehenden Ansprüche, **dadurch gekennzeichnet, dass** die Nachverfolgungseinrichtung (9) eine Kamera zum optischen Erfassen des Markers (10) und des Kontrollmarkers (11) aufweist.

6. System (3) nach einem der vorhergehenden Ansprüche, **dadurch gekennzeichnet, dass** die Nachverfolgungseinrichtung (9) und/oder der Kontrollmarker (11) zum Vorgeben ihrer räumlichen Lagebeziehung zueinander beweglich aber fixierbar an dem Roboterarm (5) befestigt sind.

7. System (3) nach einem der vorhergehenden Ansprüche, **dadurch gekennzeichnet, dass**
- das System (3) zusätzlich ein externes, insbesondere weder an dem Roboter (4) noch an dem Zielobjekt (2) befestigtes, Ensemble (12) von Hilfsmarkern (13) aufweist und
- das System (3) dazu eingerichtet ist, beim Nachverfolgen der Position des Zielobjekts (2) eine räumliche Lagebeziehung zwischen der Nachverfolgungseinrichtung (9) und dem Ensemble (12) und/oder der Hilfsmarker (13) untereinander zu messen und das Nachverfolgen der Position des Zielobjekts (2) anhand eines entsprechenden Messergebnisses zu plausibilisieren.

## Claims

1. System (3), having a robot (4) with a movable robot arm (5) and a tracking device for tracking a position of a target object (2), in particular a patient (2), on the basis of a marker (10) arranged on the target object (2) by processing data provided by the tracking device (9) and/or the marker (10) by means of a data processing device (14) of the system (3),
wherein
- the tracking device (9) is secured to the robot arm (5),
- the system (3) additionally has at least one control marker (11), and
- the system (3) is configured to measure the spatial positional relationship between the tracking device (9) and the control marker (11) by means of the tracking device (9),
**characterised in that**
- the system (3) is configured to measure the spatial positional relationship between the tracking device (9) and the control marker (11) by means of the tracking device (9) during the tracking of the position of the target object (2),
- the control marker (11) is arranged in a constant spatial positional relationship with the tracking device (9) which is specified during a movement of the robot arm (5) during the operation of the robot (4), and
- the system (3) is configured
- to compare the measured spatial positional relationship with the specified spatial positional relationship and
- in the event of a difference being identified between the measured and the specified spatial positional relationship, to generate a corresponding signal.

2. System (3) according to claim 1, **characterised in that** the control marker (11) is also secured to the robot arm (5).

3. System (3) according to one of the preceding claims, **characterised in that** the tracking device (9) is secured to a robot flange (6) or a foot (15) of the robot arm (5).

4. System (3) according to one of the preceding claims, **characterised in that** the tracking device (9) has an EM field generator and the control marker (11) has a sensor tuned to the EM field generator, in particular at least one receiver coil.

5. System (3) according to one of the preceding claims, **characterised in that** the tracking device (9) has a camera for optically detecting the marker (10) and the control marker (11) .

6. System (3) according to one of the preceding claims, **characterised in that**, to specify their spatial positional relationship with respect to one another, the tracking device (9) and/or the control marker (11) are secured movably but fixably to the robot arm (5).

7. System (3) according to one of the preceding claims, **characterised in that**
- the system (3) additionally has an external ensemble (12) of auxiliary markers (13), in particular secured to neither the robot (4) nor the target object (2), and
- the system (3) is configured to measure a spatial positional relationship between the tracking device (9) and the ensemble (12) and/or the auxiliary markers (13) with respect to one another and to check the plausibility of the tracking of the position of the target object (2) on the basis of a corresponding measurement result when tracking the position of the target object (2).

## Revendications

1. Système (3), comportant un robot (4) ayant un bras (5) de robot mobile et un dispositif de poursuite pour suivre une position d'un objet (2) cible, notamment d'un patient (2), à l'aide d'un marqueur (10) disposé sur l'objet (2) cible, par traitement, au moyen d'un dispositif (14) de traitement de données du système (3), de données procurées par le dispositif (9) de poursuite et/ou le marqueur (10),
dans lequel
- le dispositif (9) de poursuite est fixé au bras (5) du robot,
- le système (3) a, en outre, au moins un marqueur (11) de commande, et
- le système (3) est conçu pour mesurer, au moyen du dispositif (9) de poursuite, la relation de position dans l'espace entre le dispositif (9) de poursuite et le marqueur (11) de commande,
**caractérisé en ce que**
- le système (3) est conçu pour mesurer, lors du suivi de la position de l'objet (2) cible, la relation de position dans l'espace entre le dispositif (9) de poursuite et le marqueur (11) de commande, au moyen du dispositif (9) de poursuite,
- le marqueur (11) de commande est, par rapport au dispositif (9) de poursuite, dans une relation de position dans l'espace constante donnée lors d'un fonctionnement du robot (4) pour un déplacement du bras (5) du robot, et
- le système (3) est conçu pour
- comparer la relation de position dans l'espace, qui est mesurée, à la relation de position dans l'espace, qui est donnée à l'avance, et,
- s'il est détecté une différence entre la relation de position dans l'espace mesurée et celle donnée à l'avance, produire un signal correspondant.

2. Système (3) suivant la revendication 1, **caractérisé en ce que** le marqueur (11) de commande est fixé également au bras (5) du robot.

3. Système (3) suivant l'une des revendications précédentes, **caractérisé en ce que** le dispositif (9) de poursuite est fixé à un collet (6) du robot ou à un pied (15) du bras (5) du robot.

4. Système (3) suivant l'une des revendications précédentes, **caractérisé en ce que** le dispositif (9) de poursuite a un générateur de champ EM et le marqueur (11) de commande a un capteur, notamment au moins une bobine de récepteur, accordé au générateur de champ EM.

5. Système (3) suivant l'une des revendications précédentes, **caractérisé en ce que** le dispositif (9) de poursuite a une caméra de détection optique du marqueur (10) et du marqueur (11) de commande.

6. Système (3) suivant l'une des revendications précédentes, **caractérisé en ce que** le dispositif (9) de poursuite et/ou le marqueur (11) de commande sont, pour la prescription de leur relation de position dans l'espace, fixés en étant mobiles l'un par rapport à l'autre, mais en pouvant être immobilisés sur le bras (5) du robot.

7. Système (3) suivant l'une des revendications précédentes, **caractérisé en ce que**
- le système (3) a, en outre, un ensemble (12) extérieur, notamment fixé ni au robot (4) ni à l'objet (2) cible, de marqueurs (13) auxiliaires et
- le système (3) est conçu pour, lors du suivi de la position de l'objet (2) cible, mesurer une relation de position dans l'espace entre le dispositif (9) de poursuite et l'ensemble (12) et/ou les marqueurs (13) auxiliaires et apprécier la vraisemblance du suivi de la position de l'objet (2) cible à l'aide d'un résultat de mesure correspondant.
